# EUROPEAN PATENT APPLICATION

(11) **EP 3 789 488 A1**
(43) Date of publication of application: **10.03.2021**
(21) Application number: 19776395.6
(22) Date of filing: 29.03.2019
(51) Int. Cl.: C12N 5/10, C12N 5/0735

(54) **COMPOSITION THAT CONTAINS POLYLYSINE ANALOG AND PROMOTES CELL GROWTH**

(30) Priority: 30.03.2018 JP 2018069852
(71) Applicant: AJINOMOTO CO., INC., Chuo-ku Tokyo 104-8315 (JP)
(72) Inventor: HIRAMA, Ryusuke, Kawasaki-shi, Kanagawa 210-8681 (JP); KODAMA, Riho, Kawasaki-shi, Kanagawa 210-8681 (JP); KITAZAWA, Manabu, Kawasaki-shi, Kanagawa 210-8681 (JP); SUGIMOTO, Nao, Kawasaki-shi, Kanagawa 210-8681 (JP); OZAWA, Hiroki, Kawasaki-shi, Kanagawa 210-8681 (JP)
(74) Representative: Hoffmann Eitle
(86) International application number: PCT/JP2019/013992
(87) International publication number: WO 2019/189758

(57) **Abstract**

The present invention provides a composition for promoting cell proliferation containing polylysine in which at least one amino group is mono-substituted by dicarboxylic acid.

## Description

### [Technical Field]

The present invention relates to a composition for promoting cell proliferation. More specifically, the present invention relates to a composition for addition to a medium that contains a polylysine analog and is capable of promoting proliferation of stem cells and the like.

### [Background Art]

Regenerative medicine technology has made remarkable progress, and for example, transplant surgery using iPS cells was performed for the first time in the world in 2014. Regenerative medicine technology is considered to be able to provide a new treatment strategy for diseases that were difficult to treat with conventional technology, and further development is expected.

On the other hand, since regenerative medicine is a relatively new technology, it includes many problems to be solved. As one example, regenerative medicine requires large amounts of stem cells to induce tissues and organs that can be transplanted. Therefore, a technique for large-scale production of stem cell needs to be established. However, large-scale production of stem cell requires a large amount of expensive medium, which is feared to raise the medical expenses.

With this background, a stem cell culture medium using low-cost and easily-handleable components has been proposed (patent document 1).

Polylysine is roughly classified into ε-polylysine and α-polylysine according to its coupling scheme. Both are polymers in which about 15 to 35 lysines, which are one of the essential amino acids, are polymerized. In the former, the ε-amino group and α-carboxyl group present in the side chain of the lysine residue are polymerized by a peptide bond, and in the latter, the amino group at the α-position and the α-carboxyl group are polymerized by a peptide bond.

ε-Polylysine is well known as one of the food additives and is used as a preservative (patent document 2). In addition, α-polylysine is often used as a surface coating agent for culture vessels for the purpose of improving the adherence of cultured cells. Also, as an application of polylysine to cell culture technique, application to a composition for cryopreservation of cells and tissues has been reported (patent document 3).

### [Document List]

### [Patent documents]

patent document 1: JP-B-6197947
patent document 2: JP-A-2003-171462
patent document 3: WO 2009/157209

### [Summary of Invention]

### [Technical Problem]

The present invention aims to provide a novel means that can promote cell proliferation using safe and inexpensive materials.

### [Solution to Problem]

The present inventors have conducted intensive studies of the above-mentioned problems and found that proliferation of cells (particularly, stem cells) can be promoted by dissolving a polylysine analog in which the amino group of polylysine (particularly, ε-poly-L-lysine or α-poly-L-lysine) is modified with dicarboxylic acid in a medium for cell culture. Based on such finding, they have conducted further studies and completed the present invention.

That is, the present invention provides the following.
[1] A composition for promoting cell proliferation comprising polylysine in which at least one amino group is mono-substituted by dicarboxylic acid.
[2] The composition of [1], wherein the dicarboxylic acid is one kind or two or more kinds.
[3] The composition of [1] or [2], wherein the polylysine is ε-poly-L-lysine or α-poly-L-lysine.
[4] The composition of any of [1] to [3], wherein the dicarboxylic acid is any of the structures represented by the following formula III: 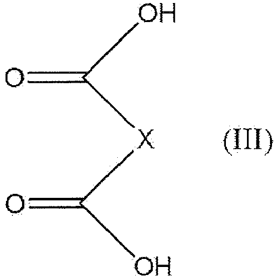 wherein X is a lower alkylene group optionally having substituent(s), a lower alkenylene group optionally having substituent(s), or -X₁-X₂-X₃-, X₁ and X₃ are each a lower alkylene group optionally having substituent(s), and X₂ is a single bond, a lower alkylene group optionally having substituent(s), S, or O.
[5] The composition of [4], wherein the dicarboxylic acid is one kind or two or more kinds selected from the group consisting of succinic acid, glutaric acid, diglycolic acid, thiodiglycolic acid, maleic acid, and citraconic acid.
[6] The composition of any of [1] to [5], wherein an introduction rate of one kind or two or more kinds of dicarboxylic acids into the amino group in polylysine is 10 mol% - 100 mol%.
[7] The composition of any of [1] to [6], wherein the cell is a stem cell.
[8] The composition of [7], wherein the stem cell is a pluripotent stem cell.
[9] The composition of [8], wherein the pluripotent stem cell is an induced pluripotent stem cell (iPS cell).
[10] A medium for cell culture, comprising the composition of any of [1] to [6].
[11] The medium of [10], wherein the cell is a stem cell.
[12] The medium of [11], wherein the stem cell is a pluripotent stem cell.
[13] The medium of [12], wherein the pluripotent stem cell is an induced pluripotent stem cell (iPS cell).
[14] A method for promoting cell proliferation, comprising culturing the cell in the medium of [10].
[15] The method of [14], wherein the cell is a stem cell.
[16] The method of [15], wherein the stem cell is a pluripotent stem cell.
[17] The method of [16], wherein the pluripotent stem cell is an induced pluripotent stem cell (iPS cell).

### [Advantageous Effects of Invention]

The present invention can prepare a large amount of cells efficiently at a low cost because it can easily promote proliferation of a cell (particularly, stem cell).

### [Brief Description of Drawings]

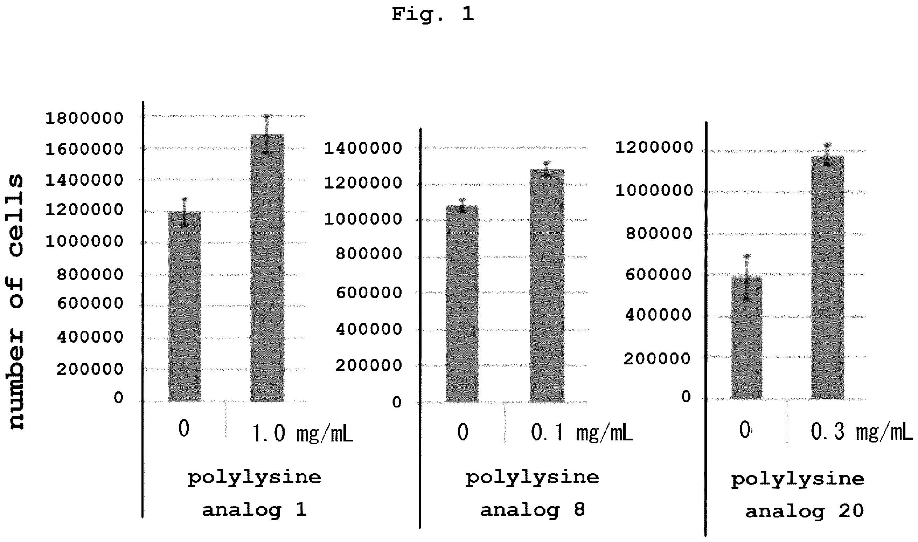

Fig. 1 shows the number of cells when iPS cells were cultured in a medium containing polylysine analog 1, 8 or 20 at a predetermined concentration.

### [Description of Embodiments]

The terms used in the present specification are defined in the following.

The "halogeno group" is fluoro, chloro, bromo, or iodo.

The "lower alkylene group" in the present specification means a straight chain or branched alkylene group having 1 - 6 carbon atoms. Specifically, groups such as methylene, ethylene, n-propylene, isopropylene, propylidene, n-butylene, sec-butylene, tert-butylene, n-pentylene, sec-pentylene, tert-pentylene, n-hexylene, sec-hexylene, tert-hexylene etc. can be mentioned.

The "lower alkenylene group" in the present specification means a straight chain or branched alkenylene group having 2 - 6 carbon atoms. Specifically, vinylene, allylene, propenylene, butenylene, prenylene, butadienylene, pentenylene, pentadienylene, hexenylene, hexadienylene and the like can be mentioned.

The "lower alkylene group" and "lower alkenylene group" may have a substituent, and examples of the substituent include the following:
(1) a halogeno group,
(2) a hydroxy group,
(3) a cyano group,
(4) a nitro group,
(5) a carboxyl group,
(6) an alkenyl group (C₂₋₁₀ alkenyl group; e.g., vinyl, allyl, propenyl, butenyl, pentenyl, hexenyl, heptenyl, butadienyl, hexatrienyl, and other respective isomers),
(7) an alkynyl group (C₂₋₁₀ alkynyl group; e.g., ethynyl, propynyl, butynyl, pentynyl, hexynyl, and other respective isomers),
(8) a halogenoalkyl group (e.g., monofluoromethyl, difluoromethyl, trifluoromethyl, monofluoroethyl, difluoroethyl, trifluoroethyl, chloromethyl, chloroethyl, dichloroethyl, and other respective isomers),
(9) a cyclic alkyl group (optionally containing hetero atom in the ring) (e.g., cyclopropyl, cyclobutyl, cyclopentyl, cyclohexyl, tetrahydrofuranyl, tetrahydropyranyl, aziridinyl, azetidinyl, pyrrolidinyl, piperidinyl, morpholinyl),
(10) an aryl group (e.g., phenyl, naphthyl),
(11) a heteroaryl group (e.g., pyridyl, pyridazinyl, pyrimidinyl, pyrazinyl, furyl, thiophenyl, pyrrolyl, pyrazolyl, imidazolyl, triazolyl (e.g., 1,2,3-triazolyl, 1,2,4-triazolyl), tetrazolyl, oxazolyl, isoxazolyl, thiazolyl, isothiazolyl, oxadiazolyl (e.g., 1,2,3-oxadiazolyl, 1,2,4-oxadiazolyl, 1,3,4-oxadiazolyl), thiadiazolyl (e.g., 1,2,3-thiadiazolyl, 1,2,4-thiadiazolyl, 1,3,4-thiadiazolyl), benzofuryl, benzothiophenyl, indolyl, isoindolyl, benzoxazolyl, benzothiazolyl, benzimidazolyl, indazolyl, benzisoxazolyl, benzisothiazolyl, benzoxadiazolyl, benzothiadiazolyl, purinyl, quinolinyl, isoquinolinyl, cinnolinyl, phthalazinyl, quinazolinyl, quinoxalinyl, pteridinyl, imidazoxazolyl, imidazothiazolyl, imidazoimidazolyl),
(12) an alkoxy group (e.g., methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy),
(13) an alkylthio group (e.g., methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio),
(14) an alkoxy group (as defined in the above-mentioned (12)) substituted by an aryl group (as defined in the above-mentioned (10)),
(15) an alkylthio group (as defined in the above-mentioned (13)) substituted by an aryl group (as defined in the above-mentioned (10)),
(16) an alkoxy group (as defined in the above-mentioned (12)) substituted by a heteroaryl group (as defined in the above-mentioned (11)),
(17) an alkylthio group (as defined in the above-mentioned (13)) substituted by a heteroaryl group (as defined in the above-mentioned (11)),
(18) a cyclic alkyl(optionally containing hetero atom in the ring)oxy group (e.g., cyclopropyloxy, cyclobutyloxy, cyclopentyloxy, cyclohexyloxy, tetrahydrofuranyloxy, tetrahydropyranyloxy, aziridinyloxy, azetidinyloxy, pyrrolidinyloxy, piperidinyloxy, morpholinyloxy),
(19) an aryloxy group (e.g., group in which an aryl group (as defined in the above-mentioned (10)) is bound to an oxygen atom),
(20) a heteroaryloxy group (e.g., group in which a heteroaryl group (as defined in the above-mentioned (11)) is bound to an oxygen atom),
(21) a halogenoalkoxy group (e.g., group in which a halogenoalkyl group (as defined in the above-mentioned (8)) is bound to an oxygen atom),
(22) a halogenoalkylthio group (e.g., group in which a halogenoalkyl group (as defined in the above-mentioned (8)) is bound to a sulfur atom),
(23) an alkoxy group (as defined in the above-mentioned (12)) substituted by a hydroxy group,
(24) an alkoxy group (as defined in the above-mentioned (12)) substituted by an alkoxy group (as defined in the above-mentioned (12)),
(25) an amino group,
(26) an amino group mono- or di-substituted by an alkyl group,
   wherein the "alkyl group" means a C₁₋₆ alkyl group can be mentioned, specifically, methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like,
(27) a carbamoyl group,
(28) a carbamoyl group mono- or di-substituted by an alkyl group (as defined for "alkyl group" in the above-mentioned (26)) (e.g., methylcarbamoyl, ethylcarbamoyl, dimethylcarbamoyl, diethylcarbamoyl, ethylmethylcarbamoyl)
(29) a sulfamoyl group,
(30) a sulfamoyl group mono- or di-substituted by an alkyl group (as defined for "alkyl group" in the above-mentioned (26)) (e.g., methylsulfamoyl, ethylsulfamoyl, dimethylsulfamoyl, diethylsulfamoyl, ethylmethylsulfamoyl),
(31) an alkanoyl group (e.g., carbonyl group in which a hydrogen atom or alkyl group (as defined for "alkyl group" in the above-mentioned (26)) is bound to a carbon atom),
(32) an aroyl group (e.g., carbonyl group in which an aryl group (as defined in the above-mentioned (10)) is bound to a carbon atom),
(33) an alkylsulfonylamino group (e.g., sulfonylamino group substituted by an alkyl group (as defined for alkyl group in the above-mentioned (26)))
(34) an arylsulfonylamino group (e.g., sulfonylamino group substituted by an aryl group (as defined in the above-mentioned (10))),
(35) a heteroarylsulfonylamino group (e.g., sulfonylamino group substituted by a heteroaryl group (as defined in the above-mentioned (11))),
(36) an acylamino group (e.g., amino group substituted by an acyl group),
   wherein the "acyl group" is an acyl group having a C₁₋₆ alkyl group or a C₆₋₁₀ aryl group, wherein the "C₁₋₆ alkyl group" is the above-mentioned "alkyl group" having 1 - 6 carbon atoms, and the "C₆₋₁₀ aryl group" is the above-mentioned "aryl group" having 6 - 10 carbon atoms, and the acyl group is specifically acetyl group, propionyl group, butyroyl group, isobutyroyl group, valeroyl group, isovaleroyl group, pivaloyl group, hexanoyl group, acryloyl group, methacryloyl group, crotonoyl group, isocrotonoyl group, benzoyl group, naphthoyl group or the like,
(37) an alkoxycarbonylamino group (e.g., carbonylamino group substituted by an alkoxy group (as defined in the above-mentioned (12))),
(38) an alkylsulfonyl group (e.g., sulfonyl group substituted by an alkyl group (as defined for "alkyl group" in the above-mentioned (26))),
(39) an alkylsulfinyl group (e.g., sulfinyl group substituted by an alkyl group (as defined for "alkyl group" in the above-mentioned (26))),
(40) an alkoxycarbonyl group (e.g., methoxycarbonyl group, ethoxycarbonyl group), and the like.

When two or more substituents are present, they may be the same or different.

### 1. Composition for promoting cell proliferation

The present invention provides a composition for promoting cell proliferation that contains polylysine in which at least one amino group is mono-substituted by dicarboxylic acid (hereinafter sometimes to be referred to as "the composition of the present invention"). In one embodiment, the polylysine in the composition of the present invention may be one kind or two or more kinds.

The polylysine used in the composition of the present invention may be any of ε-poly-L-lysine, ε-poly-D-lysine, α-poly-L-lysine, and α-poly-D-lysine, or a combination of two or more kinds of these. As the polylysine used for the composition of the present invention, ε-poly-L-lysine (formula I) or α-poly-L-lysine (formula II) is preferable. 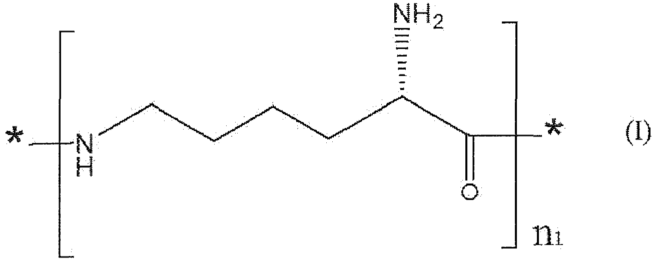 wherein n₁ is an integer of 10 - 70 (preferably 15 - 40, more preferably 20 - 40, particularly preferably 20 - 35)).

The number average molecular weight of the ε-polylysine used in the present invention is not particularly limited as long as a desired effect is obtained, and may be generally 1,000 - 20,000, preferably 1,000 - 10,000, particularly preferably 2,000 - 5,000. 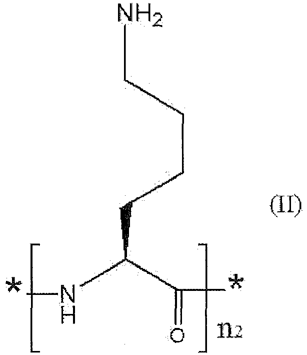 wherein n₂ is an integer of 10 - 100 (preferably 20 - 100, more preferably 20 - 80, particularly preferably 60 - 80)).

The number average molecular weight of the α-polylysine used in the present invention is not particularly limited as long as a desired effect is obtained, and may be generally 1,000 - 20,000, preferably 2,000 - 20,000, particularly preferably 7,000 - 10,000.

The polylysine used in the present invention can be produced by a method known per se, or a commercially available polylysine can also be used. Examples of the commercially available polylysine include, but are not limited to, "Poly epsilon lysine" (carbosynth Ltd. FP14985), "Poly-L-Lysine.HCl" (Wako Pure Chemical Industries, Ltd. 339-30753) and the like.

Also, the dicarboxylic acid used in the composition of the present invention is not particularly limited as long as a desired effect is obtained. In one embodiment, it includes dicarboxylic acid represented by the following formula III. 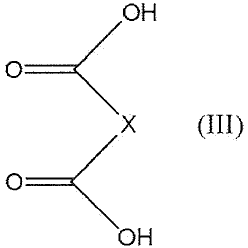 wherein X is a lower alkylene group optionally having substituent(s), a lower alkenylene group optionally having substituent (s), or -X₁-X₂-X₃-, X₁ and X₃ are each a lower alkylene group optionally having substituent(s), X₂ is a single bond, a lower alkylene group optionally having substituent(s), S, or O.

In one embodiment, dicarboxylic acid used in the composition of the present invention is dicarboxylic acid capable of forming cyclic anhydride, and specifically, may be succinic acid, glutaric acid, diglycolic acid, thiodiglycolic acid, maleic acid, or citraconic acid. In a particularly preferred embodiment of the present invention, dicarboxylic acid may be succinic acid or glutaric acid.

As shown in formula I or formula II, polylysine has one primary amino group in the side chain of the unit structure. By introducing dicarboxylic acid into the amino group through amidation, polylysine is modified (in the present specification, a polylysine having at least one amino group modified with dicarboxylic acid is sometimes to be referred to as a "polylysine analog". Therefore, the composition of the present invention can also be referred to as a "composition for promoting cell proliferation containing a polylysine analog"). The dicarboxylic acid used for modifying the polylysine may be only one kind of the aforementioned dicarboxylic acid, or two or more kinds thereof. In one embodiment, the kind of the dicarboxylic acid that modifies polylysine may be 2 kinds, 3 kinds, 4 kinds, 5 kinds, 6 kinds, 7 kinds, or 8 kinds. One kind or two or more kinds of dicarboxylic acid can be introduced into a part of the primary amino group, all primary amino groups in polylysine. In one embodiment, the introduction rate of dicarboxylic acid into polylysine is not particularly limited as long as a desired effect is obtained. It may be generally 10 mol% - 100 mol%, preferably 20 mol% - 100 mol%, more preferably 30 mol% - 90 mol%, further preferably 35 mol% - 90 mol%, particularly preferably 40 mol% - 80 mol%, with respect to the number of moles of the primary amino group in polylysine. When two or more kinds of dicarboxylic acid are used, the sum of the introduction rates of the respective dicarboxylic acids into polylysine may be set to fall within the above-mentioned range. As a method for preparing the polylysine analog used in the present invention, a method known per se can be used. Briefly, as described in detail in the Examples below, it can be prepared by mixing and heating polylysine and dicarboxylic acid anhydride, but the method is not limited thereto.

In one preferable embodiment, the polylysine analog contained in the composition of the present invention when polylysine is ε-polylysine may be polylysine modified with the kind and introduction rate of dicarbon selected from the group consisting of succinic acid (introduction rate 10 mol% - 95 mol%, preferably 20 mol% - 95 mol%, more preferably 40 mol% - 95 mol%, particularly preferably 50 mol% - 95 mol%), glutaric acid (introduction rate 10 mol% - 90 mol%, preferably 20 mol% - 90 mol%, more preferably 40 mol% - 80 mol%, particularly preferably 45 mol% - 75 mol%), diglycolic acid (introduction rate 10 mol% - 90 mol%, preferably 10 mol% - 50 mol%, more preferably 10 mol% - 30 mol%, particularly preferably 15 mol% - 30 mol%), thiodiglycolic acid (introduction rate 10 mol% - 90 mol%, preferably 20 mol% - 90 mol%, more preferably 30 mol% - 70 mol%, particularly preferably 40 mol% - 60 mol%), maleic acid (introduction rate 10 mol% - 90 mol%, preferably 20 mol% - 80 mol%, more preferably 30 mol% - 60 mol%, particularly preferably 50 mol% - 60 mol%), citraconic acid (introduction rate 10 mol% - 90 mol%, preferably 20 mol% - 90 mol%, more preferably 40 mol% - 90 mol%, particularly preferably 45 mol% - 85 mol%), a combination of succinic acid and thiodiglycolic acid (introduction rate 10 mol% - 90 mol% (total of two kinds), preferably 20 mol% - 90 mol% (total of two kinds), more preferably 40 mol% - 70 mol% (total of two kinds), particularly preferably 50 mol% - 60 mol% (total of two kinds)), and a combination of glutaric acid and citraconic acid (introduction rate 10 mol% - 90 mol% (total of two kinds), preferably 20 mol% - 90 mol% (total of two kinds), more preferably 40 mol% - 70 mol% (total of two kinds), particularly preferably 50 mol% - 60 mol% (total of two kinds)). When polylysine is α-polylysine, it may be polylysine modified by succinic acid (introduction rate 10 mol% - 90 mol%, preferably 20 mol% - 80 mol%, more preferably 30 mol% - 60 mol%, particularly preferably 50 mol% - 60 mol%).

The amount of the polylysine analog in the composition of the present invention is not particularly limited as long as a desired effect is exhibited. It may be generally 1 - 99.9 wt% of the total weight of the composition. In one embodiment, when the composition is a liquid, it may be about 1 - 5% from the aspect of solubility. When the composition is a dispersing agent, the amount may be set to 70 - 90%, but not limited thereto.

The composition of the present invention can be in any form when provided or stored. The composition can be a formulated solid such as a tablet, pill, capsule, granule, or liquid dissolved in an appropriate solvent, or in a state where it is bound to a substrate or a simple substance. Additives for formulating the composition of the present invention include antiseptics such as p-hydroxybenzoic acid esters and the like; excipients such as lactose, glucose, sucrose, mannit and the like; lubricants such as magnesium stearate, talc and the like; binders such as poly(vinyl alcohol), hydroxypropylcellulose, gelatin and the like; surfactants such as fatty acid ester and the like; plasticizers such as glycerol and the like, and the like. These additives are not limited to those described above and may be freely selected by those skilled in the art if they are utilizable.

The proliferation of cells can be promoted by culturing the cells using a cell culture medium (including a culture solution) in which the composition of the present invention is dissolved.

The composition of the present invention can be applied to any medium as long as a desired effect is afforded. In one embodiment, the composition of the present invention can be applied to DMEM, EMEM, IMDM (Iscove's Modified Dulbecco's Medium), GMEM (Glasgow's MEM), RPMI-1640, α-MEM, Ham's Medium F-12, Ham's Medium F-10, Ham's Medium F12K, Medium 199, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's MB752/1, CMRL-1066, Williams' medium E, Brinster's BMOC-3 Medium, ReproFF2 medium (ReproCELL Incorporated), StemFit (registered trade mark) AK medium (Ajinomoto Co., Inc.), TeSRTM-E6 (STEMCELL Technologies), mTeSR1 medium (STEMCELL Technologies), TeSR2 medium (STEMCELL Technologies), RHB medium (StemCells, Inc.), Improved MEM Zinc Option medium, hESF-GRO medium (Nipro Corporation), HESF-DIF medium (Nipro Corporation), CSTI-7 (Cell Science & Technology Institute, Inc.), F-12 medium, Essential 8 medium (Life Technologies), Essential 6 medium (Life Technologies, DMEM/F12 medium, BGJb medium, or mixed media of these and the like. In one preferable embodiment, the composition of the present invention can be applied to StemFit (registered trade mark) AK medium (Ajinomoto Co., Inc.), Essential 8 medium (Life Technologies), mTeSR1 medium (STEMCELL Technologies), TeSR2 medium (STEMCELL Technologies), RHB medium (StemCells, Inc.), TeSR™-E6 (STEMCELL Technologies), hESF-GRO medium (Nipro Corporation), HESF-DIF medium (Nipro Corporation), CSTI-7 (Cell Science & Technology Institute, Inc.), Essential 6 medium (Life Technologies) and the like, which are basal media for culturing pluripotent stem cells.

The amount of the composition of the present invention to the medium is not particularly limited as long as a desired effect is afforded. Generally, an amount to make the concentration of the polylysine analog in the medium 0.001 - 10 mg/mL, preferably 0.005 - 5 mg/mL, further preferably 0.01 - 3 mg/mL, particularly preferably 0.03 - 3 mg/mL can be added. The concentration of the polylysine analog in the medium can be appropriately changed in consideration of the culture conditions, culture form (e.g., adhesion culture or suspension culture) and the like.

The type of cell that shows promoted proliferation by culturing using a medium in which the composition of the present invention is dissolved is not limited. The cell is preferably a stem cell, more preferably a pluripotent stem cell.

In the present specification, the "pluripotent stem cell" is an immature cell having self-replication ability and differentiation/proliferation ability and capable of differentiating into all tissues and cells constituting a living body. Examples of the pluripotent stem cell include embryonic stem cells (ES cell), induced pluripotent stem cell (iPS cell) (Takahashi K et al., Cell. 2007 Nov 30; 131(5):861-72), spermatozoon stem cell (mGS cell) (Kanatsu-Shinohara M et al., Biol Reprod. 2007 Jan; 76(1):55-62), embryonic germ cell (Matsui Y et al., Cell. 1992 Sep 4; 70(5):841-7) and the like.

Pluripotent stem cell can be obtained by a method known per se. For example, in the case of embryonic stem cell (ES cell), a method for culturing inner cell mass in mammalian blastocyst (e.g., the method described in Manipulating the Mouse Embryo:A Laboratory Manual, Fourth Edition 2014 Cold Spring Harbor Laboratory Press), a method for culturing early embryo produced by somatic cell nuclear transfer (Wilmut et al., Nature. 1997 Feb 27; 385(6619):810-3, Wakayama et al., Nature. 1998 Jul 23; 394(6691):369-74, Wakayama T et al., Science. 2001 Apr 27; 292(5517):740-3) and the like; however, the method is not limited to these.

Further, embryonic stem cell can also be obtained from a given institution. For example, human ES cells KhES-1 cell, KhES-2 cell and KhES-3 cell are available from Institute for Frontier Medical Sciences, Kyoto University.

Examples of the method for obtaining induced pluripotent stem cell (iPS cell) include, but are not limited to, a method for introducing a nuclear reprogramming substance (e.g., Oct3/4, Sox2, c-Myc and Klf4 etc.) into somatic cells (Takahashi K et al., Cell. 2006 Aug 25; 126(4):663-76, WO 2007/069666). In addition, induced pluripotent stem cells can be produced according to the methods described in Takahashi K et al., Nat Protoc. 2007; 2(12):3081-9, Aoi et al., Science. 2008 Aug 1; 321(5889):699-702, Takahashi, K et al., Cell. 2007 Nov 30; 131(5):861-72, Yu, J et al., Science. 2007 Dec 21; 318(5858):1917-20, Nakagawa, M et al., Nat Biotechnol. 2008 Jan; 26(1):101-6, and the like; however, the method is not limited to these.

Further, induced pluripotent stem cell can also be purchased from a given institution. For example, human iPS cells 253G1 cell, and 201B7 cell are available from iPS Academia Japan, Inc.

Embryonic germ cell can be induced by isolating primordial germ cells according to a conventional method and culturing them in the presence of LIF, bFGF and SCF. In addition, mGS cells can be produced from testis cells according to the method described in WO 2005/100548.

Pluripotent stem cell is preferably an embryonic stem cell or induced pluripotent stem cell (iPS cell), more preferably induced pluripotent stem cell (iPS cell).

These pluripotent stem cells are derived generally from mammals. Examples of the mammal include, but are not limited to, rodents such as mouse, rat, hamster, guinea pig and the like, Lagomorpha such as rabbit and the like, ungulates such as swine, bovine, goat, horse, sheep and the like, carnivora such as dog, cat and the like, primates such as human, monkey, Macaca mulatta, marmoset, orangutan, chimpanzee and the like, and the like. Pluripotent stem cells derived from rodents such as mouse and the like or primates such as human and the like are preferable, pluripotent stem cell derived from human is more preferable, and human induced pluripotent stem cell is most preferable.

The conditions for cell culture using a medium in which the composition of the present invention is dissolved may be appropriately set based on a method known per se. For example, the culture temperature is not particularly limited as long as a desired effect such as promotion of cell proliferation and the like can be achieved. It is about 30 - 40°C, preferably about 37°C. The CO₂ concentration is about 1 - 10%, preferably about 2 - 5%. The oxygen concentration is generally 1 - 40%, and appropriately selected according to the culture conditions and the like. As the culture form, any of adhesion culture, suspension culture, embedded culture, and tissue culture may be used.

### 2. Medium for cell culture

The present invention also provides a medium for cell culture containing the composition of the present invention (hereinafter sometimes to be referred to as "the medium of the present invention").

In one embodiment, the medium of the present invention is prepared by dissolving a polylysine analog in a medium for cell culture. The medium for cell culture may be a known or commercially available medium. The known or commercially available medium, and the concentration of the polylysine analog to be dissolved in the medium are the same as those described in the "Composition of the present invention".

The form of the medium of the present invention is not particularly limited as long as a desired effect is obtained. For example, it can be prepared in the form of a liquid medium, a semi-solid medium, or a solid medium. Also, the medium of the present invention may be prepared in a powder form. By preparing a powder form, transportation and storage may be facilitated. Such powder medium can be conveniently made into a liquid medium by adding sterilized water or the like immediately before use and sterilizing as necessary using a membrane filter or the like. The medium of the present invention can be used in any culture form such as adhesion culture, suspension culture, embedded culture, tissue culture, and the like.

The kind of cells in which the proliferation is promoted by the medium of the present invention is the same as that described in the "Composition of the present invention".

### 3. Cell culture method

The present invention also provides a method for promoting cell proliferation by culturing cells in the medium of the present invention (hereinafter sometimes to be referred to as "the method of the present invention").

The method of the present invention may be a method for promoting cell proliferation by culturing cells in a medium containing a polylysine analog dissolved therein.

The existing medium for cell culture used in the method of the present invention, the concentration of the polylysine analog dissolved in the medium, the kind of cells in which the proliferation is promoted, the conditions for cell culture, and the like are the same as those described in the "Composition of the present invention".

The present invention is explained more specifically in the following Examples. The present invention is not limited at all by these examples.

### [Example]

### [Example 1] Preparation of polylysine analogs

Various polylysine analogs were prepared by the methods described below.

### Polylysine analog 1

ε-Poly-L-lysine hydrochloride (Carbosynth Ltd. FP14985) (2.0 g, 12.2 mmol per Lys unit) was dissolved in 30 mL of water, 30 mL of a basic ion exchange resin (Amberlite (registered trade mark), IRA- 400 washed with an alkali to give a free form) was added, and the mixture was shaken and allowed to stand for 1 hr. The resin was filtered off using a Kiriyama funnel, the filtrate was concentrated under reduced pressure, and the obtained residue was dissolved in 10 mL of water. Succinic anhydride (726 mg, 7.26 mmol, 0.6 equivalents per Lys unit) was added, and the mixture was stirred at 60°C for 2 hr. After allowing to cool, the mixture was lyophilized to give 2.12 g of a succinic acid-modified product of ε-poly-L-lysine as a white solid.

¹H-NMR(D₂O, 400 MHz) δ= 1.12-1.53(76.6, m), 1.53-1.90(42.1,m), 2.36-2.62(53.5,m),2.845-3.00(2.55,m), 3.00-3.30(36.5,m), 3.60-3.74(1.0,m), 3.74-3.97(5.5,m), 3.98-4.23(12.4,m)

Regarding the ¹H-NMR broad peak around 3.8 ppm as the acyl group-unintroduced lysine residue α-position proton, and the broad peak around 4.1 ppm as the acylated lysine residue α-position proton, the acylation introduction rate was calculated. acyl group introduction rate (polylysine analog 1)=0.69

### Polylysine analogs 2 - 19, 30, 31

Using the kinds and amounts of cyclic acid anhydrides shown in Table 1 below, polylysine analogs 2 - 19, 30, 31 were prepared. For polylysine analogs 2 - 19, the reaction conditions were similar to the conditions used for the preparation of polylysine analog 1; for polylysine analogs 30, 31, 20-mer ε-poly-L-lysine TFA salt (11.6 mmol per Lys unit) that can be synthesized by a known method was used as the starting material instead of ε-poly-L-lysine hydrochloride (Carbosynth Ltd. FP14985, 12.2 mmol per Lys unit). Where necessary, dialysis was performed with a MwCO1000Da dialysis tube before lyophilizing. The acyl group introduction rate was also calculated in the same manner.

**[Table 1]**

| poly lysine analog | kind of dicarboxylic acid anhydride | amount of dicarboxylic acid anhydride used (molar equivalent per lysine unit) | acyl group introduction rate |
|---|---|---|---|
| 2 | succinic anhydride | 0.4 | 0.51 |
| 3 | | 0.5 | 0.56 |
| 4 | | 0.7 | 0.74 |
| 5 | | 0.8 | 0.93 |
| 6 | | 0.9 | 0.86 |
| 7 | glutaric anhydride | 0.4 | 0.45 |
| 8 | | 0.6 | 0.63 |
| 9 | | 0.8 | 0.72 |
| 10 | diglycolic anhydride | 0.4 | 0.19 |
| 11 | | 0.6 | 0.26 |
| 12 | | 0.8 | 0.23 |
| 13 | thiodiglycolic anhydride | 0.4 | 0.43 |
| 14 | | 0.6 | 0.51 |
| 15 | | 0.8 | 0.56 |
| 16 | maleic anhydride | 0.4 | 0.52 |
| 17 | citraconic anhydride | 0.4 | 0.47 |
| 18 | | 0.6 | 0.66 |
| 19 | | 0.8 | 0.83 |
| 30 | succinic anhydride | 0.6 | 0.58 |
| 31 | glutaric anhydride | 0.6 | 0.68 |

### Polylysine analog 20

α-Poly-L-lysine hydrochloride (Wako Pure Chemical Industries, Ltd. 339-30753) (1.0 g, 6.1 mmol per Lys unit) was dissolved in a mixture (10 mL) of water:acetonitrile=1:1, sodium hydrogen carbonate (613 mg), succinic anhydride (365 mg, 0.6 equivalents per Lys unit) were successively added, and the mixture was stirred at 60°C for 2 hr. After air cooling, the reaction mixture was dialyzed for 2 days while exchanging the outer liquid (MWCO=1000), and the resulting solution was lyophilized to give 906 mg of α-poly-L-lysine succinic acid modified product as a white solid (acyl group introduction rate=0.60).

### Polylysine analogs 21 and 22

Using the kinds and amounts of cyclic acid anhydrides shown in Table 2 below, polylysine analogs 21 and 22 were prepared. The reaction conditions were similar to those used in the preparation of the polylysine analog 1. They were synthesized by dissolving 0.5 - 1 g of the modified product in 20 mL of water, neutralizing the solution to pH7 with 1 M-NaOH, and lyophilizing the solution.

**[Table 2]**

| polylysine analog | kind of dicarboxylic acid anhydride | amount of dicarboxylic acid anhydride used (molar equivalent per lysine unit) | acyl group introduction rate (total of two kinds) |
|---|---|---|---|
| 21 | succinic anhydride/ thiodiglycolic anhydride | 0.25/ 0.25 | 0.54 |
| 22 | glutaric anhydride/ citraconic anhydride | 0.25/ 0.25 | 0.58 |

### [Example 2] Cell proliferation promoting effect 1 of polylysine analogs (evaluation of number of colonies formed after culture)

The polylysine analogs prepared in Example 1 were dissolved in a cell culture medium, and the cell proliferation promoting effect thereof was confirmed.

More particularly, the 201B7 strain purchased from iPS Academia Japan, Inc. was used as the iPS cell. The cells were cultured using a culture container (24 well cell culture plate manufactured by Nippon Becton Dickinson, Ltd.) coated with vitronectin (Life Technologies Japan Ltd.) under the conditions of 5% CO₂, 37°C. A test compound aqueous solution was added to Essential 8 medium (manufactured by Invitrogen) to a given concentration and used as a test medium. This was used for culture immediately or after storage at 4°C for 2 - 4 weeks, and the effect thereof was studied. The iPS cell suspension was seeded at 600 cells/well. Y-27632 was added to the medium used at the time of seeding (final concentration 10 µM, manufactured by Nacalai Tesque: 08945-84). On the second day after seeding, the medium was replaced with a test medium not containing Y-27632. Three days after the seeding, the state of the adhered cells was photographed at 9 fields/well (area 0.57 cm²) of phase difference images with a fluorescent microscope BZ-X700 (KEYENCE CORPORATION) in accordance with the manual. The number of colonies in each well was measured using the analysis software attached to the above-mentioned device, and compared with the number of colonies formed when the same culture was performed without adding the test compound.

As regards activity intensity, "++" indicates formation of 3 times or more colonies, and "+" indicates formation of 1.2 times or more colonies, than in the polylysine analog non-addition group. In addition, the addition concentrations of the polylysine analogs are also shown. The results are shown in the following Table 3.

**[Table 3]**

| polylysine analog | activity intensity | evaluation concentration [mg/mL] |
|---|---|---|
| 1 | ++ | 1 |
| 2 | ++ | 0.3 |
| 3 | + | 3 |
| 4 | ++ | 0.3 |
| 5 | + | 0.3 |
| 6 | + | 1 |
| 7 | ++ | 0.3 |
| 8 | ++ | 1 |
| 9 | ++ | 1 |
| 10 | + | 0.03 |
| 11 | ++ | 0.03 |
| 12 | + | 0.1 |
| 13 | ++ | 0.1 |
| 14 | ++ | 1 |
| 15 | + | 0.3 |
| 16 | + | 0.3 |
| 17 | + | 0.3 |
| 18 | + | 1 |
| 19 | ++ | 0.3 |
| 20 | ++ | 1 |
| 21 | + | 0.3 |
| 22 | + | 0.3 |
| 30 | + | 0.3 |
| 31 | + | 0.3 |

### [Example 3] Cell proliferation promoting effect 2 of polylysine analogs (evaluation of number of cells after culture)

The 201B7 strain purchased from iPS Academia Japan, Inc. was used as the iPS cell. The cells were cultured using a culture container (Nippon Becton Dickinson Company, Falcon culture dish or Falcon culture plate) coated with vitronectin (Life Technologies Japan Ltd.) under the conditions of 5% CO₂, 37°C. An aqueous solution of polylysine analog 1, 8 or 20 was added to Essential 8 medium (manufactured by Invitrogen) to a given final concentration and used as a test medium. This was used for culture immediately or after storage at 4°C for 2 - 4 weeks, and the effect thereof was studied. Y-27632 was added to the medium used at the time of seeding (final concentration 10 µM, manufactured by Nacalai Tesque: 08945-84). From the next day, the cells were cultured in a test medium not containing Y-27632. The medium was replaced every 2 to 3 days, and after culturing for 6 days, the number of cells was measured. The cells were counted by the method described in "Revised: Introduction to Cell Culture, pages 77-83, 2010, YODOSHA CO., LTD.", and compared with the number of cells when the same culture was performed without adding the test compound. The results are shown in Fig. 1.

### [Example 4] Cell proliferation promoting effect 3 of polylysine analogs (evaluation of number of colonies formed after culture)

An experiment was conducted under the same conditions as in Example 2 except that StemFit (registered trade mark) AK02 was used instead of Essential 8, and the cell proliferation promoting effect of the polylysine analog was confirmed. As regards activity intensity, "++" indicates formation of 3 times or more colonies, and "+" indicates formation of 1.2 times or more colonies, than in the polylysine analog non-addition group. In addition, the addition concentrations of the polylysine analogs are also shown. The results are shown in the following Table 4.

**[Table 4]**

| polylysine analog | activity intensity | evaluation concentration [mg/mL] |
|---|---|---|
| 2 | + | 0.3 |
| 5 | + | 0.3 |
| 8 | + | 0.3 |
| 9 | + | 0.3 |

### [Industrial Applicability]

The present invention can prepare a large amount of cells (particularly, stem cells) efficiently at a low cost, and thus is extremely beneficial in the field of regenerative medicine.

This application is based on a patent application No. 2018-069852 filed in Japan (filing date: March 30, 2018), the contents of which are incorporated in full herein.

## Claims

1. A composition for promoting cell proliferation comprising polylysine in which at least one amino group is mono-substituted by dicarboxylic acid.

2. The composition according to claim 1, wherein the dicarboxylic acid is one kind or two or more kinds.

3. The composition according to claim 1 or 2, wherein the polylysine is ε-poly-L-lysine or α-poly-L-lysine.

4. The composition according to any one of claims 1 to 3, wherein the dicarboxylic acid is any of the structures represented by the following formula III: 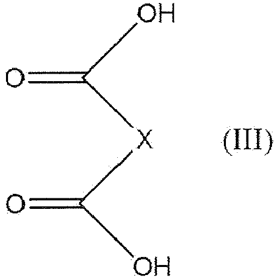 wherein X is a lower alkylene group optionally having substituent(s), a lower alkenylene group optionally having substituent (s), or -X₁-X₂-X₃-, X₁ and X₃ are each a lower alkylene group optionally having substituent(s), and X₂ is a single bond, a lower alkylene group optionally having substituent(s), S, or O.

5. The composition according to claim 4, wherein the dicarboxylic acid is one kind or two or more kinds selected from the group consisting of succinic acid, glutaric acid, diglycolic acid, thiodiglycolic acid, maleic acid, and citraconic acid.

6. The composition according to any one of claims 1 to 5, wherein an introduction rate of one kind or two or more kinds of dicarboxylic acids into the amino group in polylysine is 10 mol% - 100 mol%.

7. The composition according to any one of claims 1 to 6, wherein the cell is a stem cell.

8. The composition according to claim 7, wherein the stem cell is a pluripotent stem cell.

9. The composition according to claim 8, wherein the pluripotent stem cell is an induced pluripotent stem cell (iPS cell).

10. A medium for cell culture, comprising the composition according to any one of claims 1 to 6.

11. The medium according to claim 10, wherein the cell is a stem cell.

12. The medium according to claim 11, wherein the stem cell is a pluripotent stem cell.

13. The medium according to claim 12, wherein the pluripotent stem cell is an induced pluripotent stem cell (iPS cell).

14. A method for promoting cell proliferation, comprising culturing the cell in the medium according to claim 10.

15. The method according to claim 14, wherein the cell is a stem cell.

16. The method according to claim 15, wherein the stem cell is a pluripotent stem cell.

17. The method according to claim 16, wherein the pluripotent stem cell is an induced pluripotent stem cell (iPS cell).
